# EUROPEAN PATENT APPLICATION

(11) **EP 4 144 392 A1**
(43) Date of publication of application: **08.03.2023**
(21) Application number: 21195076.1
(22) Date of filing: 06.09.2021
(51) Int. Cl.: A61M 11/00, A61M 15/00, C08L 67/04

(54) **AEROSOLTHERAPY DEVICE COMPRISING A HOUSING MADE OF BIOPOLYMERIC MATERIAL**

(71) Applicant: Air Liquide Medical Systems S.R.L., 20158 Milano (IT)
(72) Inventor: RUOCCO, Alessandra, 25073 Bovezzo (IT); SANDONI, Giuseppe, 25073 Bovezzo (IT); ALBERICI, Luca, 25073 Bovezzo (IT)
(74) Representative: Air Liquide

(57) **Abstract**

The invention deals with an aerosoltherapy device comprising an external housing for protecting inner components, such as a motor/compressor unit and a piping system. The external housing is partially or totally made of biopolymeric material obtained from vegetal material.

## Description

The present invention concerns an aerosoltherapy device comprising an external housing for protecting inner components, such as a motor/compressor unit, a piping system or other elements, said external housing being made of biopolymeric, i.e. bioplastic or biopolymer, material obtained from vegetal material.

An aerosoltherapy device generally comprises a peripheral rigid body or casing, usually called "external housing", that protects the inner components of the device, such as an electric motor/compressor unit, piping systems and/or other elements.

The aerosoltherapy device provides compressed gas, typically ambient air, to another device, typically a nasal washing device or a nebulizer device that is used for nebulizing a liquid, such as a washing solution or a drug-containing solution having suitable sizes for reaching the lower airways of the patient that has to inhale the thus obtained nebulized solution, i.e. a medication-containing mist, in the frame of his/her aerosoltherapy.

The gas, in particular ambient air, is compressed by the motor/compressor unit that sucks ambient air and delivers it after compression to a piping system, i.e. gas passages, conduits, lines or the like, that convoys the compressed or pressurized gas. Examples of aerosoltherapy devices are given by EP-A-3678719, EP-A-3027255 and WO-A-2011/161706.

The compressed gas is then conveyed to the other device, namely a nasal washing device or a nebulizer device, by means of a flexible hose of the like.

A nebulizer delivers an aerosolized drug-containing solution, i.e. a medication-containing mist, to the patient's airways, by means of a patient interface, such as a nasal mask, a mouth piece or similar, that provides the gaseous drug to the patient. Examples of nebulizer devices useable with an aerosoltherapy device are given by EP-A-3459577, EP-A-3441097, EP-A-34410978, WO-A-03/053500 and GB-A-2358356.

Further, a nasal washing device provides a washing solution to the nasal parts of a user, i.e. the nostrils, by means of a nasal cannula or the like, in the aim of cleaning them, as taught by EP-A-2732881 or EP-A-1044699.

Today, plastic materials are commonly used for manufacturing some parts, especially the peripheral external housing, of aerosoltherapy devices. Indeed, plastic materials are cheap, easy to manufacture and to transform, and exhibit a good robustness/resistance to shocks and similar.

Using plastic materials is however not satisfying when taking into account environmental considerations as plastic materials are one of the biggest sources of greenhouse gas emissions, contributing to the global pollution and likely to global warming. Indeed, plastic materials are generally produced from oil, i.e. fossil fuels.

In this contect, a problem is to provide an aerosoltherapy device using less plastic materials for limiting its negative environmental impact, without decreasing its robustness and its resistance to shocks or the like.

A solution according to the present invention concerns an aerosoltherapy device comprising an external housing comprising at least one inner component, characterized in that at least part of the external housing is made of at least one biopolymeric material, i.e. a bioplastic material or the like, obtained from and/or containing at least one vegetal material.

Depending on the embodiment, the aerosoltherapy device of the present invention can comprise one or several of the following additional features:
- the external housing forms the peripheral body of the aerosoltherapy device.
- the external housing is rigid.
- the external housing comprises a bottom wall, a top wal and lateral walls, in particular 4 lateral walls.
- the lateral walls are arranged between the bottom wall and a top wall, and attached thereto, i.e. the lateral walls project from the bottom wall toward the top wall and are attached to said wall and a top wall.
- the external housing delimits an internal volume comprising said at least one inner component.
- said at least one inner component is chosen from a motor/compressor unit and a piping system, or any other element.
- said at least one biopolymeric material contains at least 20 w.% of vegetal material, preferably at least 25 w.%.
- said at least one biopolymeric material contains at least 30 w.% of vegetal material, preferably at least 35 w.%
- said at least one biopolymeric material contains at least 40 w.% of vegetal material.
- said vegetal material comprises at least one extract or derivate of cereales, such as corn or wheat, or of potatoe, tapioca or sugarcane.
- said at least one extract or derivate comprises starch, preferably corn or potatoe starch.
- said at least one biopolymeric material is polylactic acid (PLA).
- said biopolymeric material is polylactic acid (PLA) obtained from corn starch.
- alternatively, said at least one biopolymeric material is chosen among Bio-PVC, Bio-PA, Bio-PP, Bio-PC, Bio-PE, Bio-PETand Bio-ABS.
- said biopolymeric material is bio-PET composed of about 70% of terephtalic acid (PTA) and of about 30% of monoethylen glycol (MEG).
- the external housing comprises at least two shell-elements attached one to another.
- the shell-elements are made of said at least one biopolymeric material.
- the external housing further comprises a cover made of said at least one biopolymeric material.
- the cover is detachably-attached to the external housing.
- the cover is pivotably-attached to the external housing.
- the external housing further comprises at least one inner wall made of said at least one biopolymeric material.
- the external housing comprises an upper shell-element and a lower shell-element attached together.
- the upper shell-element and the lower shell-element are attached together along a contact border comprising two parallelly-arranged portions, preferably straight or curved portions, i.e. border sections.
- the two parallelly-arranged portions define a contact plane forming an angle of about between 30° and 60 ° with a vertical plane, i.e. the vertical direction.
- the upper shell-element and the lower shell-element are separated one from the other by the contact plane.
- the upper shell-element comprises an inner compartment comprising an inner bottom.
- the upper shell-element further comprises the cover, preferably a pivotally-attached cover.
- the upper shell-element further comprises an enlarged opening normally closed by the cover and through which the user can access the inner compartment.
- the inner compartment is used for storing a flexible hose(s), a nebulizer device, a nasal washing device or any other instrument.
- the lower shell-element comprises the motor/compressor unit.
- a power source, like a battery, a mains connection or the like, is arranged in the housing.
- the power source provides electrical power to the motor/compressor unit.

Further, the invention also concerns a process for manufacturing the external housing of an aerosoltherapy device according to the present invention, namely as described hereabove, wherein at least one biopolymeric material obtained from or containing at least one vegetal material is used for making at least part of the external housing, i.e. the structure of the external housing comprises said at least one biopolymeric material, such as PLA.

Preferably, the making at least part of the external housing comprises molding a least a part of said external housing.

An embodiment of an aerosoltherapy device according to the present invention is shown in the enclosed Figures, among which:
- Figure 1A shows an embodiment of an aerosoltherapy installation comprising an aerosoltherapy device fluidly coupled to a nebulizer device,
- Figure 1B shows an alternative embodiment of Figure 1A, wherein the aerosoltherapy device is fluidly coupled to a nasal washing device,
- Figures 2 and 3 are different views of an embodiment of an aerosoltherapy device according to the present invention,
- Figures 4 is an exploded view of the aerosoltherapy device of Figures 2-3,
- Figures 5 shows the cover of the aerosoltherapy device of Figures 2-3, and
- Figures 6 shows an embodiment of a motor/compressor unit arranged in the aerosoltherapy device of Figures 2-3.

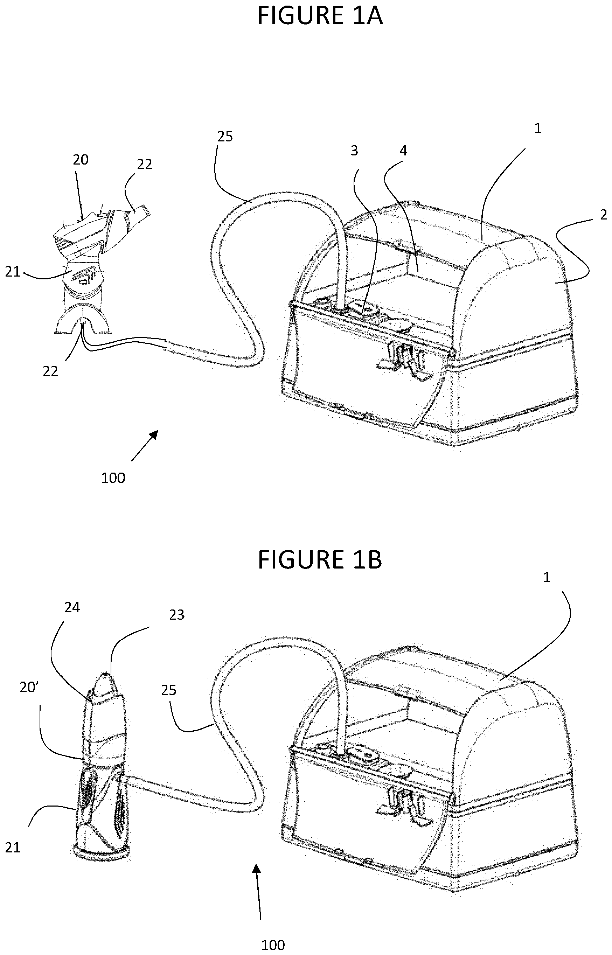

Figure 1A illustrates an aerosoltherapy installation 100 comprising an aerosoltherapy device 1 fluidly coupled to nebulizer device 20 useable for treating a patient, i.e. a human being, in need of an aerosoltherapy treatment, and Figure 1B is similar to Figure 1A, except that the aerosoltherapy device 1 is fluidly coupled to nasal washing device 20' useable for cleaning the nasal parts, i.e. nostrils, of a user.

The aerosoltherapy device 1 of Figures 1A-1B comprises an external rigid housing 2 or casing, that constitutes a peripheral body that protects its inner components, in particular the electric motor/compressor unit 3, the inner piping systems and other elements, for instance electrical connections and/or wires, an ON/OFF button, a silencer, a filter... The external housing 2 is made of a rigid material.

It further comprises a power source, such as a battery, a mains connection or the like, for providing electrical power to the motor/compressor unit 3 or any other component requiring electrical power for working.

In use, the motor/compressor unit 3 sucks ambient air, compresses it and delivers the compressed air thus obtained, via a flexible hose 25, to another device 20, 20', namely a nasal washing device 20' (in Fig. 1B) or a nebulizer device 20 (in Fig. 1A), that uses said compressed air for nebulizing/aerosolizing a liquid solution, such as a nasal cleaning solution (in Fig. 1B) or a drug-containing solution (in Fig. 1A), i.e. a liquid medication or drug, respectively.

In Fig. 1A, the nebulizer device 20 comprises a main body 21 that can be handhold by the user, i.e. the patient, a hose connector 22 for fluidly connecting thereto the flexible hose 25 that conveys compressed air, and a patient respiratory interface 23, such as a mouthpiece, a nasal mask, nasal prongs/cannulas or the like. The main body 21 of the nebulizer device 20 further comprises a reservoir or tank for storing the liquid-drug solution and an aerosol-generating system for generating an aerosol, i.e. a mist, comprising droplets of liquid drug carried by the compressed air flow. The aerosol-generating system can further comprise a deflector, such as a blade, and an injector, such as nozzle with an opening, as taught by EP-A-3459577 or EP-A-3441097. Gas conducts or passages are arranged into the main body 21 of the nebulizer device 20 for conveying the fluids, namely compressed air, liquid drug and/or aerosol mist. The patient respiratory interface 23 delivers the aerosol mist that contains droplets of liquid drug/medication of suitable sizes to the patient's airways, while the patient inhales the thus obtained nebulized or aerosolized solution, i.e. the medication-containing mist, in the frame of his/her aerosoltherapy or treatment.

In Fig. 1B, the nasal washing device 20' comprises a main body 21 that can be also handhold by the user, including a pressurized air injector, a liquid tank for storing the washing liquid to be atomized, an atomizing chamber for aerosolizing the washing solution by means of compressed air and inner conduit(s) or passage(s) for conveying compressed air and the aerosolized solution. The nasal washing device 20' further comprises a patient respiratory interface 23, such as a nasal nozzle or the like, for delivering the aerosolized washing solution to the nostrils of the user. A waste-recovery chamber 24 is arranged as a sleeve around the nasal nozzle and is used for recovering nasal-cleaning wastes, such as mucus, washing-solution wastes or the like.

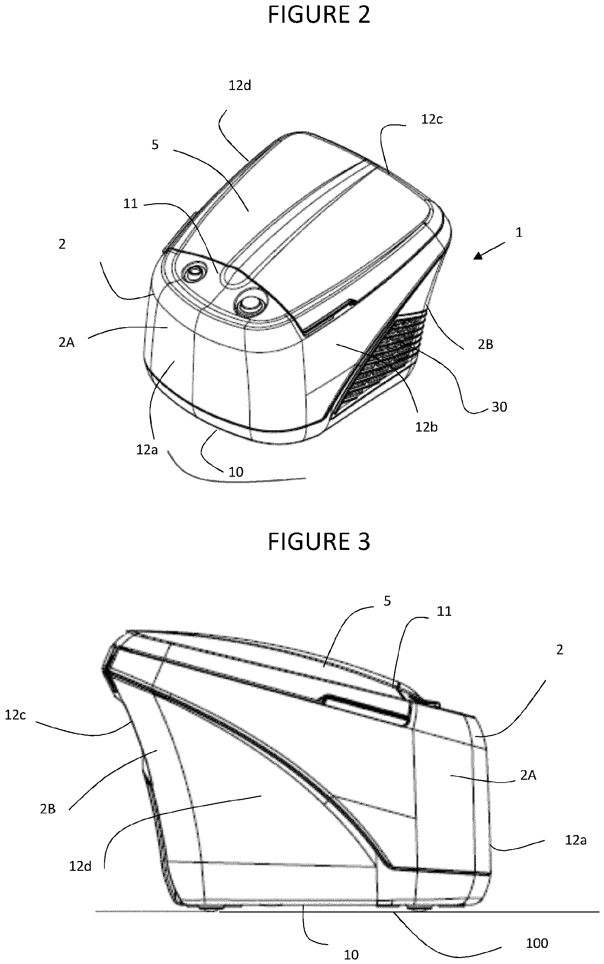

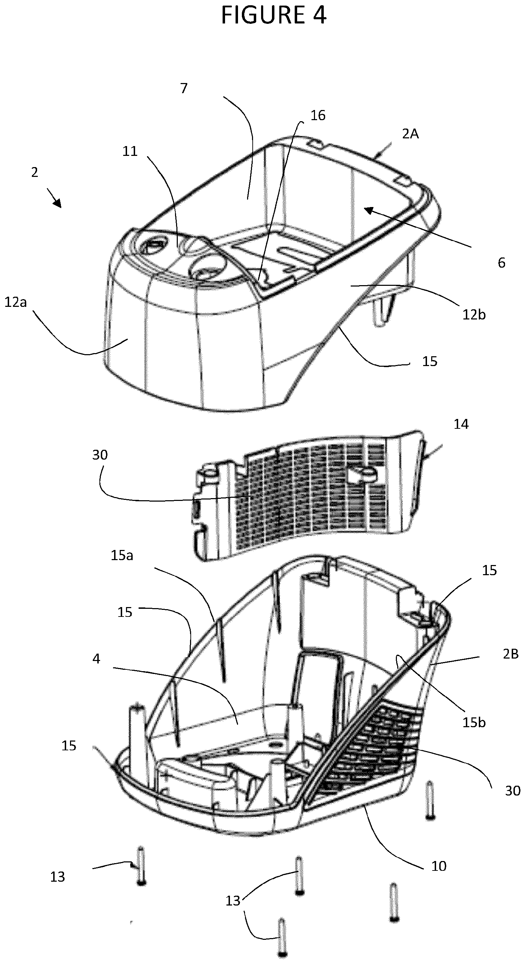

Figures 2 and 3 are a 3/4 high view (Fig. 2) and a side view (Fig. 3), whereas Figure 4 is an exploded view of an embodiment of an aerosoltherapy device 1 according to the present invention, that can be used in an aerosoltherapy installation 100 as, for instance, the one illustrated in Figures 1A and 1B.

As one can see on Fig. 2-4, the aerosoltherapy device 1 according to the present invention is similar to the one of Figures 1A-1B. It comprises an external housing or casing 2 that forms the rigid peripheral body of the aerosoltherapy device 1.

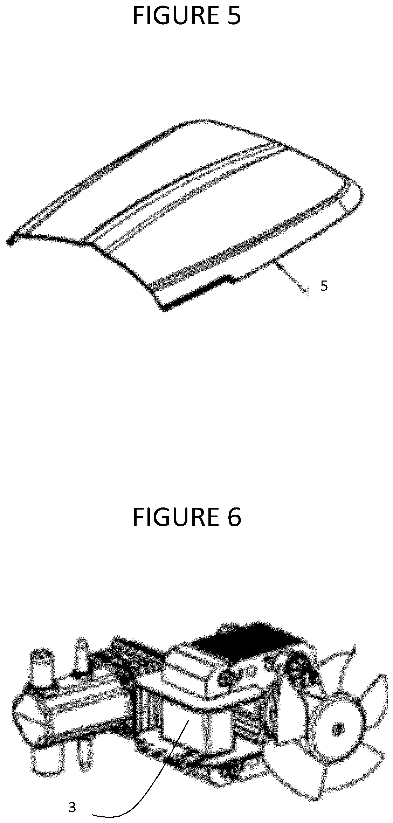

Housing 2 defines an inner hollow volume or compartment 4 containing the inner components of the device 1, in particular the motor/compressor unit 3 as shown in Figure 6, a piping system and other elements, as stated hereabove.

More precisely, the housing 2 comprises a bottom wall 10, a top wal 11 and four lateral walls 12a-12d arranged between the bottom wall 10 and the top wal 11, i.e. projecting upwardly from the bottom wall 10 toward the top wal 11, and attached thereto.

The bottom wall 10, top wal 11 and four lateral walls 12a-12d form the peripheral walls of the rigid housing 2 of the device 1 and further delimit the internal hollow volume 4 of the housing 2 comprising said inner components, in particular the motor/compressor unit 3 of Figure 6.

In the embodiment shown in Figures 2-4, the rigid housing 2 is formed of 2 sub-units 2A, 2B coupled together, namely an upper shell-element 2° (i.e. upper sub-unit) and a lower shell-element 2B (i.e. lower sub-unit) attached together as shown in Figure 4. Preferably, the 2 sub-units 2A, 2B are secured together by means of screw elements 13 or the like.

Preferably, the upper and lower shell-elements 2A, 2B are fixed together along contact border 15 comprising two parallelly-arranged portions 15a, 15b that can be straight or slightly curved as shown in Figure 4. Those portions 15a, 15b define a junction plane or the like that is angled with respect to the vertical direction, preferably of an angle of about between 30° and 60 ° with a vertical plane.

The bottom wall 10 forms the underside/lower surface of the housing 2, which faces the support 100, such as the ground, a table or any other support, on which sits the device 1 as shown in Figure 3.

Further, top wal 11 can comprise a detachable or mobile, preferably pivotable, cover 5 as shown in Figure 5. Once the cover 5 is removed or pivoted, a enlarged opening 6 gives access to an inner storing compartment 7 arranged in housing 2, in particular in the upper shell-element 2A, that can be used for storing the flexible hose 25 and/or the nebulizer device 20 of Figure 1A, or the nasal washing device 20' of Figure 1B, when not used, and/or any other instruments that might be useful for the user.

The inner storing compartment 7 arranged in the upper shell-element 2A comprises a base wall 16 forming the bottom of said upper shell-element 2A.

Preferably, each sub-unit 2A, 2B is made as one piece, for instance by injection molding or the like.

According to the present invention, at least a part of the external housing 2, such as the sub-units 2A, 2B, is made of a biopolymeric material, i.e. a bioplastic material, obtained from and/or containing vegetal material(s), preferably at least 20%, more preferably at least 30 w% to 40 w% of vegetal material, such as extract or derivate of cereales (e.g. corn, wheat or others), potatoe, tapioca or sugarcane, such as starch, preferably corn or potato starch.

Generally speaking, it is advocated to use biopolymers with a high content of renewable resources, such as corn starch, tapioca roots, chips or starch, or sugarcane for instance.

A suitable biopolymer is for instance polylactic acid (PLA) that is generally produced from corn starch. Indeed, PLA is a durable bio-polymer exhibiting good mechanical characteristics, such as strength and density, and thus suitable to produce parts of the housing 2 of an aerosol therapy device 1 meant to be used for many years.

Of course, other biopolymers can be used in lieu of PLA, such as bio-PVC (PVC =polyvinyl chloride), bio-PA (PA=polyamid), bio-PP (PP=polypropylen), bio-PC (PC=polycarbonate), bio-PE (PE=polyethylen), bio-PET (PET=polyethylen terephtalate) or bio-ABS (ABS =acrylonitrile butadiene styrene), that are produced from or contain plant extracts, derivates or the like.

Thus, bio-PET for instance is composed of about 70% of terephtalic acid (PTA) and of about 30% of monoethylen glycol (MEG) produced from vegetal materials, such as molasses of sugar canes. The final product is equivalent to regular PET in terms of properties but is 100% recyclable.

Preferably, the parts of the external housing 2 made of a biopolymeric material comprise the two sub-units 2A, 2B, namely the upper shell-element 2A and the lower shell-element 2B that are attached together, the cover 5 of Figure 5 and preferably any inner wall(s) 14 arranged into the two sub-units 2A, 2B.

More preferably, the external housing 2 and/or the inner wall(s) 14 can comprise aeration structures or grids 30, especially the lower shell-element 2B, for evacuating at least a part of the heat generated by the electric motor/compressor unit 3.

Generally speaking, the parts of the external housing 2 that can be made of a biopolymeric material are the parts that are not in contact with the patient or the user.

Thanks to the present invention the global CO₂ emission due to the production of the aerosol therapy device is lower as biopolymers with a high content of renewable resources are used as raw material for the production of the desired parts of the external housing 2.

## Claims

1. Aerosoltherapy device (1) comprising an external housing (2) comprising at least one inner component (3), **characterized in that** at least part of the external housing (2) is made of at least one biopolymeric material obtained from or containing at least one vegetal material.

2. Aerosoltherapy device according to Claim 1, **characterized in that** said at least one biopolymeric material contains at least 20 w% of vegetal material, preferably at least 30 w%, more preferably at least 40 w.% of vegetal material.

3. Aerosoltherapy device according to Claims 1 or 2, **characterized in that** said vegetal material comprises at least one extract or derivate of cereales, such as corn or wheat, or of potatoe, tapioca or sugarcane.

4. Aerosoltherapy device according to Claim 3, **characterized in that** said at least one extract or derivate comprises starch, preferably corn or potatoe starch.

5. Aerosoltherapy device according to Claim 1 or 2, **characterized in that** said at least one biopolymeric material is polylactic acid.

6. Aerosoltherapy device according to Claim 1 or 2, **characterized in that** said at least one biopolymeric material is chosen among Bio-PVC, Bio-PA, Bio-PP, Bio-PC, Bio-PE, Bio-PET and Bio-ABS.

7. Aerosoltherapy device according to Claim 1, **characterized in that** the external housing (2) comprises at least two shell-elements (2A, 2B) attached one to another, said shell-elements (2A, 2B) being made of said at least one biopolymeric material.

8. Aerosoltherapy device according to Claim 1, **characterized in that** the external housing (2) further comprises a cover (5) and/or at least one inner wall (14), made of said at least one biopolymeric material.

9. Aerosoltherapy device according to Claims 1 or 7, **characterized in that** the external housing (2) comprises an upper shell-element (2A) and a lower shell-element (2B) attached together along a contact border (15) comprising two parallelly-arranged portions (15a, 15b).

10. Aerosoltherapy device according to Claim 9, **characterized in that** the upper shell-element (2A) comprises an inner compartment (7) comprising an inner bottom (16), and the cover (5).

11. Aerosoltherapy device according to Claim 1, **characterized in that** said at least one inner component is chosen from a motor/compressor unit (3) and a piping system.

12. Aerosoltherapy device according to Claims 9 and 11, **characterized in that** the lower shell-element (2B) comprises a motor/compressor unit (3).

13. Aerosoltherapy device according to Claim 7, **characterized in that** the two shell-elements (2A, 2B) are secured together by screw elements (13).

14. Aerosoltherapy device according to Claim 8, **characterized in that** the cover (5) is pivotally-attached to the external housing (2), preferably to the upper shell-element (2A).

15. Process for manufacturing the external housing (2) of an aerosoltherapy device (1) according to anyone of the previous claims, characterized at least one biopolymeric material obtained from or containing at least one vegetal material is used for making at least part of the external housing (2).
